# EUROPEAN PATENT SPECIFICATION

(11) **EP 2 403 851 B1**
(45) Date of publication and mention of the grant of the patent: **17.06.2015**
(21) Application number: 10726656.1
(22) Date of filing: 03.03.2010
(51) Int. Cl.: C07D 451/10

(54) **NEW CRYSTALLINE FORMS OF TIOTROPIUM BROMIDE**
NEUE KRISTALLINE FORMEN VON TIOTROPIUMBROMID
NOUVELLES FORMES CRISTALLINES DE BROMURE DE TIOTROPIUM

(30) Priority: 06.03.2009 TR 200901728
(43) Date of publication of application: 11.01.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000048
(87) International publication number: WO 2010/101538

(56) References cited:
- WO-A2-2006/117299
- WO-A2-2006/117300

## Description

### Field of the invention

Present invention relates to new crystal forms of an active agent, preparation processes of these forms, pharmaceutical compositions containing these forms and these compositions for use in the treatment of respiratory disorders.

### Background of the invention

Present invention relates to new crystal forms of tiotropium bromide, preparation processes of these forms, pharmaceutical compositions containing these forms and these compositions for use in the treatment of respiratory disorders.

Tiotropium (Formula I), is an anticholinergic agent with a chemical name (1α, 2β, 4β, 5α, 7β)-7-[(hydroxidi-2-thienylacetyl)oxy]-9,9-dimethyl-3-oxa-9-azoniatricyclo [3.3.1.0^{2,4}] nonane.

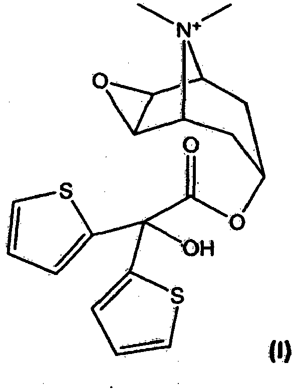

Tiotropium, is described in patent application EP0418716 (A1) (USRE39820 E1, US5610163 A ve WO9104252 A1 existing in its patent family) for the first time. The application relates to processes for preparing tiotropium forms, pharmaceutical compositions containing them, long-acting, strong anticholinergic activity of tiotropium and use of it in the treatment of respiratory disorders.

Tiotropium is a long acting, strong anticholinergic bronchodilator, which is administered orally by dry powder inhalation for the treatment of respiratory disorders. Tiotropium antagonizes the effect of acetylcholine by blocking cholinergic muscarinic receptors. Tiotropium is separated slowly from M1 and M3 receptors causing broncho-construction, but separated rapidly from M2 receptors inhibiting release of acetylcholine from cholinergic nerve endings. This situation occured in lung receptors demonstrates long acting bronchodilator activity of the drug.

Inhalation route is a commonly preferred treatment method for respiratory disorders especially chronic disorders such as asthma and chronic obstructive pulmonary disorder (COPD) which become threatening widely in the society. The reason is that drug reaches directly and rapidly to target area; by comparison with doses required for use via oral or parenteral route, lower doses show desired effect because of delivering the drug to target area directly; and drug which is used in lower doses, show less side-effect than the drug administered via oral and parenteral route shows. Gastrointestinal disturbances, such as low resolution, low permeability, drug irritation, production of undesired metabolites and decrease of bioavailability depending on food, are felt in minimum level because drugs administered via inhalation route are not exposed to gastrointestinal medium.

In addition to advantages of said inhalation treatment, there are various difficulties that should be considered during converting active ingredient to a form suitable for use by inhalation. For example, active agent administered by inhalation should meet optimally essential requirements, such as appropriate aerodynamic particle size, appropriate particle shape, uniformity of particle size distribution, low aerodynamic dispersion forces, low density, high physical and chemical stability. Among these features, appropriate particle size, uniformity of particle size distribution and physical and chemical stability are related to structural properties of the active ingredient.

"Stability" generally means that stability of the active ingredient showed both in environmental conditions, and during production of the formulation and also after -converted into the final product. The reason of why concept of stability have come into prominence, is the importance of that the amount of active agent delivered to target area have not to be less than required amount because of administering low dose. For example, moisture absorption, which is one of the factors affecting physical stability, will cause active ingredient being lower than required amount. Another example is tendency in the direction of changing polymorphic structure, which is one of the factors affecting chemical stability and is ain undesirable situation. Active ingredient should protect its crystalline form under mechanical effects, heat effect etc. and it should have no change in its polymorphic structure.

Appropriate particle size and uniformity of particle size distribution are also closely related to structural characteristics of the active ingredient. Since particles having only a certain size (1-10µm, preferably <5µm) can be transferred to the lungs via inhalation, required amount of a powder mixture containing particles of different size will be unable to reach to target area. While large particles accumulate in areas outside the target area, small particles reach target area as desired and are absorbed there. Particle size have to be decreased to required size for providing homogenization of particle size distribution, in other words, uniformity of particle size, to obtain the desired efficiency. For this purpose, difficult steps like micronization are applied to powder mixture comprising active ingredient. In the meantime, active ingredient should have a stable structure allowing micronization and should protect its crystalline form under mechanical effect.

In conclusion, new forms (e.g. solvates) of the active ingredient having better structural features which can meet said requirements, should be produced. Since each form of the same molecule exhibits different characteristics, if there are many alternative forms for choosing suitable one for the treatment by inhalation, the possibility of reaching the best result will be high.

Various documents of tiotropium forms are available in the prior art. For example, WO0230928 A1 describes crystalline monohydrate form of tiotropium bromide and WO03000265 A1 describes crystalline anhydrate form of tiotropium bromide.

WO2006/117299 A2 describes crystalline anhydrate form of tiotropium bromide, its crystalline 1,4-dioxan solvate, crystalline ethanol solvate, crystalline methanol solvate, crystalline anisol solvate, crystalline n-butanol solvate, crystalline N,N-dimethylacetamide solvate, crystalline N,N-dimethylformamide solvate, crystalline isopropanol solvate, crystalline 1,2-propandiole solvate, crystalline pyridine solvate, crystalline tert-butanol solvate, crystalline tetrahydrofuran solvate and crystalline tetrahydropyrane solvate and crystalline tetrahydropyran solvate.

WO 2006/117300 A2 describes crystalline anhydrate form of tiotropium bromide, crystalline methanol solvate, crystalline ethanol solvate, crystalline isopropanol solvate, crystalline tetrahydrofuran solvate, crystalline 1,4-dioxan solvate, crystalline dimethylformamide solvate, crystalline methylene chloride/methyl ethyl ketone mixture solvate and crystalline 1-butanol solvate.

Said solvates are prepared by starting from monohydrate or anhydrate form of tiotropium bromide. However, when preparation processes of solvates are examined, it is seen that used techniques are insufficient in respect of providing solvates having better structural properties. For example, degradation of its monohydrate form under vacuum takes place at a temperature in the range of 60 to 90°C, this demonstrates that thermal stability is low.

Eventually, according to the prior art, various forms of tiotropium are produced. However, it is seen that these forms are insufficient on having better structural properties to meet requirements directed to transmission by inhalation route. For this reason, production of new forms having better structural properties is still needed.

### Summary of the invention

The present invention relates to new crystalline allyl alcohol solvate, crystalline furfuryl alcohol solvate and crystalline 1,4-butandiole solvate of tiotropium bromide.

In one aspect, the present invention provides preparation processes of novel crystalline forms of tiotropium bromide.

In another aspect, the present invention provides pharmaceutical compositions containing novel crystalline forms of tiotropium bromide and their preparation processes.

In another aspect, the present invention provides pharmaceutical compositions containing novel crystalline forms of tiotropium bromide suitable for invention, for use in the treatment of respiratory disorders, especially asthma and COPD.

### Detailed description of the invention

Different forms can be obtained depending on the selection of solvents and process conditions during crystallization process of the active ingredients. Studies demonstrate that tiotropium bromide can be also produced in different forms.

The present invention relates to novel crystalline forms -especially solvates- of tiotropium bromide. According to the present invention solvates are obtained by using preferably alcohol as solvent. Since solvates used in the present invention, have been already containing solvent, moisture absorption was minimized; thereby, essential factor, that can be a problem for physical stability with regard to the structure of the active ingredient, was controlled. Moreover, solvates used in the invention have provided the minimization of the degradation tendency of polymorphic structure under the mechanical and thermal effect, which is an essential factor that can be a problem for chemical stability with regard to the structure of the active ingredient.

The present invention relates to crystalline allyl alcohol solvates of tiotropium bromide, preparation processes of said solvates, pharmaceutical compositions containing said solvates and the use of said composition for the treatment of respiratory disorders.

The present invention relates to crystalline furfuryl alcohol solvates of tiotropium bromide, preparation processes of said solvates, pharmaceutical compositions containing said solvates and said compositions for use in the treatment of respiratory disorders.

The present invention relates to crystalline 1,4 butandiol solvates of tiotropium bromide, preparation processes of said solvates, pharmaceutical compositions containing said solvates and said compositions for use in the treatment of respiratory disorders.

### Structural determination of solvates in accordance with the present invention

### Analysis methods used for structural determination of solvates in accordance with the present invention

### • NMR analysis method

NMR spectrums are obtained by using device with following characteristics.

### Characteristics of NMR spectrometer:

Brand name: Varian
Model: Unity Inova 500 MHz

Probe type wherein measurements are made:
500 Mhz 1H-19F(15N-31P) 5mm PFG Switchable Probe

### Parameters of proton measurements:

tn = ¹H (499.66MHz) (observed core and its frequency),
d1=1s (waiting time between pulses),
nt=256 (pulse number),
sw=7994.4 Hz (spectral width),
np= 32K (number of data points),
at=1.892s (data receiving time),
Solvent: D20, 99.9%

### • X-ray powder diffraction analysis method

X-ray powder diffraction patterns have been obtained by the device Bruker D8 Advance using Copper Kα source. X-ray tube has been worked with a current of 40 mA and a potential difference of 40 kV. Obtained X-ray powder diffraction patterns have been restricted by 2θ angles in the range of 5 to 40°. Step range is determined as 2θ=0.02°, and step duration is determined as 1 second. Samples were spun by 30 cycles per minute.

### • DSC analysis method

DSC thermograms are obtained by using the device WETZSCH DSC204. Measurement points of 800 or 1200 (800 for allyl, 1200 for the others) per minute was taken with the device. Heating rate was adjusted to 20°C/min. Amount of analyzed sample was determined as 2 mg, analyses was made in the drilled aluminum sample cup.

### Brie description of figures:

Figure 1: NMR spectrum of crystalline allyl alcohol solvate of tiotropium bromide
Figure 2: X-ray powder diffraction pattern of crystalline allyl alcohol solvate of tiotropium, bromide
Figure 3: DSC thermogram of crystalline allyl alcohol solvate of tiotropium bromide
Figure 4: NMR spectrum of crystalline furfuryl alcohol solvate of tiotropium bromide
Figure 5: X-ray powder diffraction pattern of crystalline furfuryl alcohol solvate of tiotropium bromide
Figure 6: DSC thermogram of crystalline furfuryl alcohol solvate of tiotropium bromide
Figure 7: NMR spectrum of crystalline 1,4-butandiol solvate of tiotropium bromide
Figure 8: X-ray powder diffraction pattern of crystalline 1,4-butandiol solvate of tiotropium bromide
Figure 9: DSC thermogram of crystalline 1,4-butandiol solvate of tiotropium bromide
Figure 10: NMR spectrum of tiotropium bromide anhydrate

### Datas in respect of structure determination of solvates in accordance with the invention and evaluation

### • Crystalline allyl alcohol solvate of tiotropium bromide

### - NMR analysis results

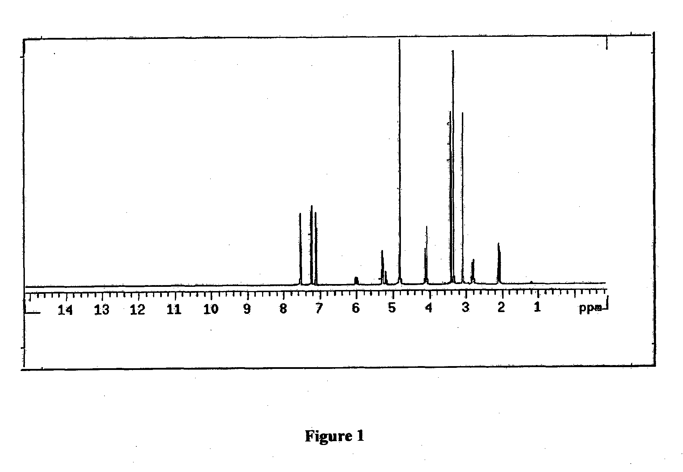

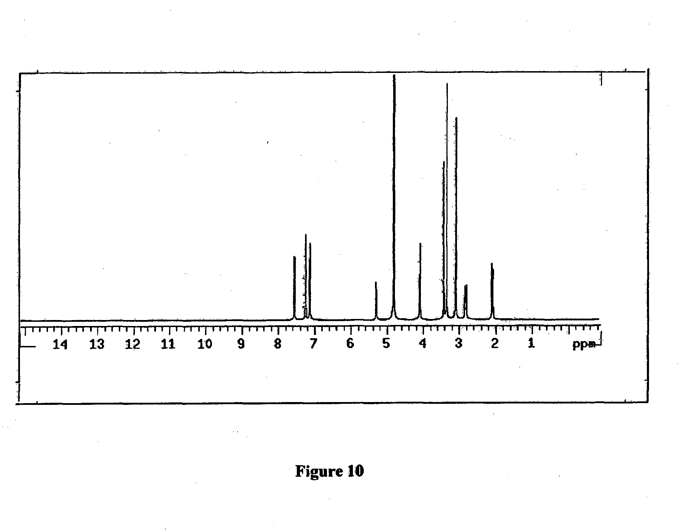

NMR spectrum of crystalline allyl alcohol solvate of tiotropium bromide is given in Figure 1. When NMR datas of said solvates and of anhydrate form of tiotropium bromide (Figure 10) are compared with each other, it is seen that peaks belonging to the solvent in crystalline form of the crystalline allyl alcohol solvate are not obtained in anhydrate form of tiotropium bromide. Different peaks come from the solvate. Anhydrate form of tiotropium bromide is obtained by drying of solvates at 160°C in accordance with the invention. This situation is a sign of high thermal stability of solvates.

According to analysis results, obtained solvate is allyl alcohol hemisolvate. Evaluation is made by comparing solvent peak integrals with active ingredient peak integrals.

### - X-ray powder diffraction analysis results

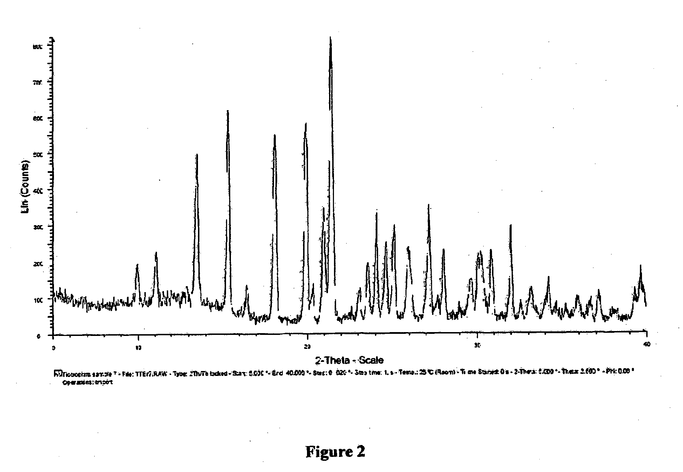

X-ray powder diffraction pattern of crystalline allyl alcohol solvates of tiotropium bromide is given in Figure 2. Peak values of crystalline allyl alcohol solvates of tiotropium bromide are placed in Table 1.

**Table 1. Peak Values of crystalline allyl alcohol solvates of tiotropium bromide**

| 2θ(°) | d(Å) | Intensity | Intensity (%) |
|---|---|---|---|
| 9,936 | 8,89476 | 193 | 23,5 |
| 11,056 | 7,99646 | 225 | 27,4 |
| 12,905 | 6,85464 | 128 | 15,6 |
| 13,465 | 6,5707 | 433 | 52,8 |
| 15,346 | 5,76906 | 617 | 75,2 |
| 16,433 | 5,38982 | 130 | 15,9 |
| 18,106 | 4,89565 | 549 | 67 |
| 19,921 | 4,45338 | 580 | 70,7 |
| 20,311 | 4,36869 | 138 | 16,8 |
| 20,975 | 4,23194 | 346 | 42,2 |
| 21,409 | 4,14708 | 820 | 100 |
| 22,601 | 3,93106 | 72 | 8;8 |
| 23,054 | 3,85484 | 119 | 14,5 |
| 23,572 | 3,77117 | 193 | 23,5 |
| 24,072 | 3,69406 | 349 | 42,6 |
| 24,632 | 3,61135 | 257 | 31,3 |
| 25,095 | 3,5457 | 298 | 36,3 |
| 25,954 | 3,43021 | 236 | 28,8 |
| 27,173 | 3,27911 | 354 | 43,2 |
| 27,662 | 3,22224 | 103 | 12,6 |
| 28,02 | 3,18181 | 238 | 29 |
| 28,974 | 3,07922 | 84 | 10,2 |
| 29,625 | 3,01301 | 149 | 18,2 |
| 30,08 | 2,96847 | 218 | 26,6 |
| 30,858 | 2,89538 | 222 | 27,1 |
| 31,987 | 2,7957 | 296 | 36,1 |
| 32,576 | 2,74648 | 91 | 11,1 |
| 33,173 | 2,69846 | 125 | 15,2 |
| 33,47 | 2,67518 | 78 | 9,5 |
| 34,203 | 2,61948 | 153 | 18,7 |
| 34,667 | 2,58547 | 67 | 8,2 |
| 35,236 | 2,54503 | 79 | 9,6 |
| 35,931 | 2,49736 | 93 | 11,3 |
| 36,673 | 2,44852 | 97 | 11,8 |
| 37,165 | 2,41725 | 115 | 14 |
| 38,08 | 2,36123 | 63 | 7,7 |
| 39,318 | 2,28969 | 122 | 14,9 |
| 39,657 | 2,27091 | 181 | 22,1 |

According to analysis results, crystalline allyl alcohol solvate of tiotropium bromide is characterized by giving peaks at a 2θ angle of 21.4 ± 0.2° in X-ray powder diffraction pattern.

### - DSC analysis results

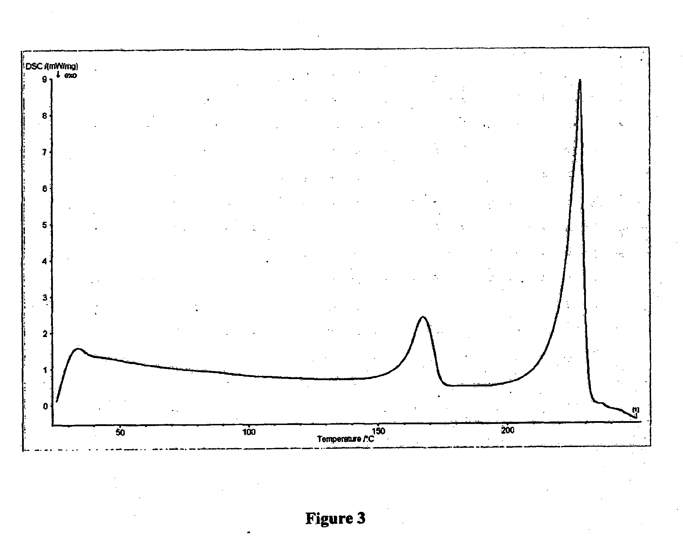

DSC thermogram of crystalline allyl alcohol solvate of tiotropium bromide is given in Figure 3.

According to the analysis results, crystalline allyl alcohol solvate of tiotropium bromide is characterized by giving an endotermic peak in the range of 130 to 180°C in DSC thermogram.

### • Crystalline furfuryl alcohol solvate of tiotropium bromide

### - NMR analysis results

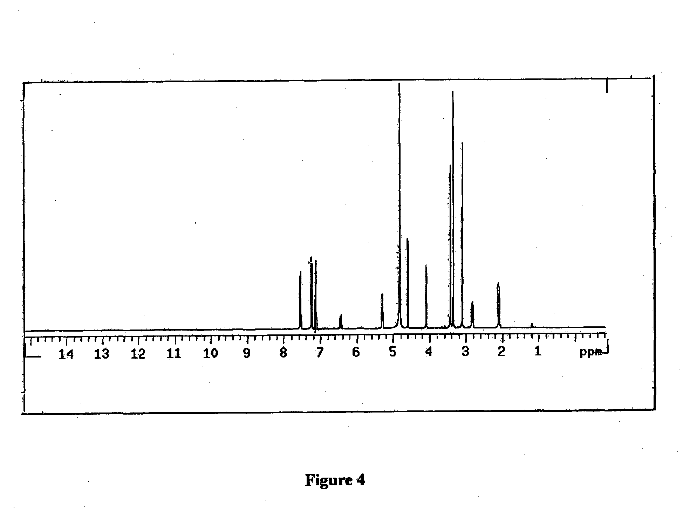

NMR spectrum of crystalline furfuryl alcohol solvate of tiotropium bromide is given in Figure 4. When NMR datas of said solvates and of anhydrate form of tiotropium bromide (Figure 10) are compared with each other, it is seen that peaks belonging to the solvent in crystalline form of the crystalline furfuryl alcohol solvate are not obtained in anhydrate form of tiotropium bromide. Different peaks come from the solvate. Anhydrate form of tiotropium bromide is obtained by drying of solvates at 160°C in accordance with the invention. This situation is a sign of high thermal stability of solvates.

According to analysis results, obtained solvate is furfuryl alcohol hemisolvate. Evaluation is made by comparing solvent peak integrals with active ingredient peak integrals.

### - X-ray powder diffraction analysis results

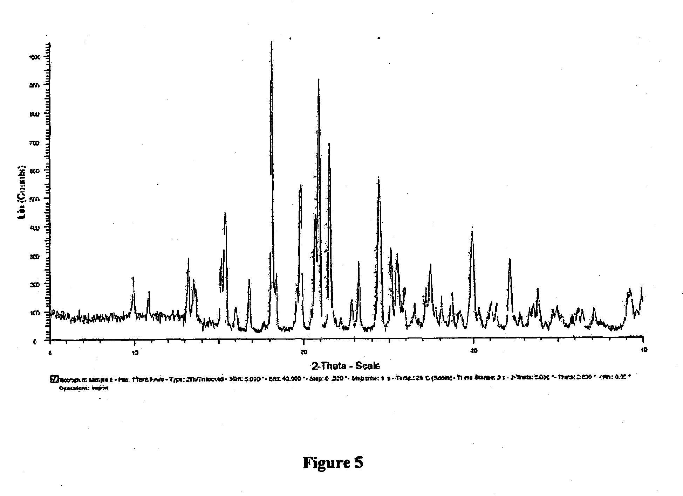

X-ray powder diffraction pattern of crystalline furfuryl alcohol solvates of tiotropium bromide is given in Figure 5. Peak values of crystalline furfuryl alcohol solvates of tiotropium bromide are placed in Table 2.

**Table 2. Peak values of crystalline furfuryl alcohol solvates of tiotropium bromide**

| 2θ(°) | d(Å) | Intensity | Intensity (%) |
|---|---|---|---|
| 9,92 | 8,90963 | 221 | 21,1 |
| 10,817 | 8,17244 | 170 | 16,3 |
| 13,202 | 6,70104 | 288 | 27,5 |
| 13,528 | 6,54004 | 229 | 21,9 |
| 15,153 | 5,84231 | 285 | 27,2 |
| 15,396 | 5,75068 | 446 | 42,6 |
| 16,042 | 5,52028 | 110 | 10,5 |
| 16,832 | 5,26309 | 216 | 20,7 |
| 17,661 | 5,01793 | 62 | 5,9 |
| 18,154 | 4,88264 | 1046 | 100 |
| 18,4 | 4,81795 | 229 | 21,9 |
| 19,58 | 4,53018 | 164 | 15,7 |
| 19,821 | 4,47564 | 544 | 52 |
| 20,7 | 4,28753 | 439 | 42 |
| 20,915 | 4,24388 | 917 | 87,7 |
| 21,546 | 4,12109 | 689 | 65,9 |
| 22,171 | 4,00631 | 73 | 7 |
| 22,815 | 3,89454 | 135 | 12,9 |
| 23,23 | 3,82597 | 283 | 27,1 |
| 24,454 | 3,63722 | 542 | 51,8 |
| 25,142 | 3,53915 | 320 | 30,6 |
| 25,499 | 3,49039 | 299 | 28,6 |
| 25,908 | 3,43625 | 178 | 17 |
| 26,508 | 3,35981 | 122 | 11,7 |
| 27,183 | 3,27786 | 179 | 17,1 |
| 27,456 | 3,24592 | 268 | 25,6 |
| 27,76 | 3,21107 | 109 | 10,4 |
| 28,116 | 3,17118 | 149 | 14,2 |
| 28,726 | 3,10527 | 155 | 14,8 |
| 29,195 | 3,05645 | 95 | 9,1 |
| 29,936 | 2,98245 | 392 | 37,5 |
| 30,341 | 2,94351 | 105 | 10 |
| 31,042 | 2,87862 | 128 | 12,2 |
| 31,365 | 2,8497 | 121 | 11,6 |
| 32,165 | 2,78067 | 275 | 26,3 |
| 32,782 | 2,72973 | 88 | 8,4 |
| 33,356 | 2,68405 | 103 | 9,8 |
| 33,543 | 2,66948 | 133 | 12,7 |
| 33,853 | 2,64577 | 136 | 14,9 |
| 34,736 | 2,58048 | 101 | 9,7 |
| 34,952 | 2,56502 | 112 | 10,7 |
| 35,206 | 2,5471 | 81 | 7,7 |
| 35,849 | 2,50288 | 80 | 7,6 |
| 36,185 | 2,4804 | 102 | 9,8 |
| 36,454 | 2,46275 | 99 | 9,5 |
| 37,154 | 2,41791 | 110 | 10,5 |
| 39,211 | 2,29571 | 172 | 16,4 |

According to analysis results, crystalline furfuryl alcohol solvate of tiotropium bromide is characterized by giving peaks at a 2θ angle of 18.2 ± 0.2° in X-ray powder diffraction pattern.

### - DSC analysis results

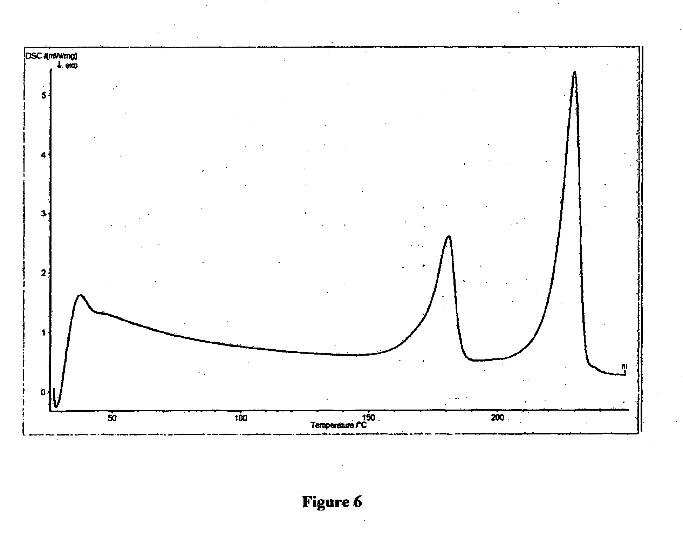

DSC thermogram of crystalline furfuryl alcohol solvate of tiotropium bromide is given in Figure 6.

According to analysis results, crystalline furfuryl alcohol solvate of tiotropium bromide is characterized by giving an endotermic peak in the range of 150 to190°C in DSC thermogram.

### • Crystalline 1,4-butandiol solvate of tiotropium bromide

### - NMR analysis results

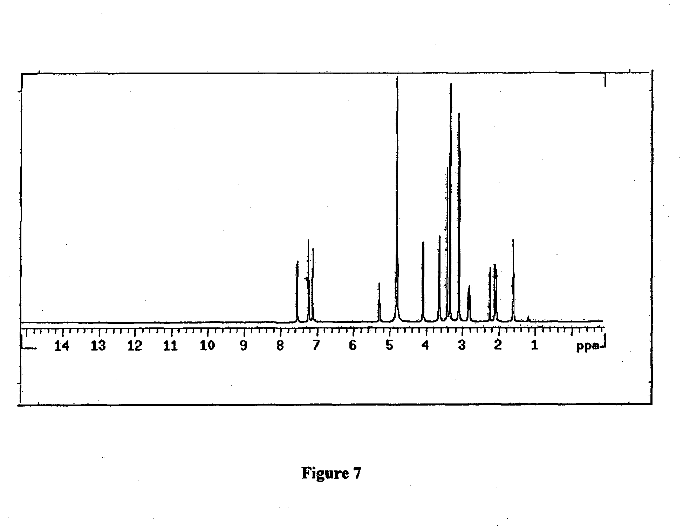

NMR spectrum of crystalline 1,4-butandiol solvate of tiotropium bromide is given in Figure 7. When NMR datas of said solvates and of anhydrate form of tiotropium bromide (Figure 10) are compared with each other, it is seen that peaks belonging to the solvent in crystalline form of the crystalline 1,4-butanediol solvate are not obtained in anhydrate form of tiotropium bromide. Different peaks come from the solvate. Anhydrate form of tiotropium bromide is obtained by drying solvates at 160°C in accordance with the invention. This situation is a sign of high thermal stability of solvates.

According to analysis results, obtained solvate is 1,4-butandiole solvate, wherein the proportion of butanediol/tiotropium bromide is ¾. Evaluation is made by comparing solvent peak integrals with active ingredient peak integrals.

### - X-ray powder diffraction analysis results

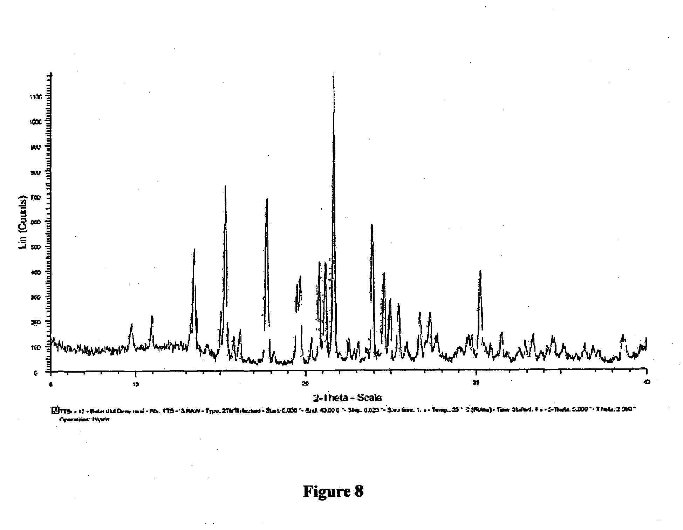

X-ray powder diffraction pattern of crystalline 1,4-butandiol solvates of tiotropium bromide is given in Figure 8. Peak values of crystalline 1,4-butandiol solvates of tiotropium bromide are placed in Table 3.

**Table 3. Peak values of crystalline 1,4-butanediol solvates of tiotropium bromide**

| 2θ (°) | d (Å) | Intensity | Intensity (%) |
|---|---|---|---|
| 9,751 | 9,06358 | 188 | 15,8 |
| 10,943 | 8,07865 | 220 | 18,5 |
| 13,456 | 6,575 | 487 | 40,9 |
| 14,244 | 6,21288 | 107 | 9 |
| 15,02 | 5,89368 | 238 | 20 |
| 15,289 | 5,79042 | 737 | 61,8 |
| 15,788 | 5,60852 | 106 | 8,9 |
| 16,147 | 5,48477 | 152 | 12,8 |
| 16,675 | 5,31243 | 53 | 4,4 |
| 17,722 | 5,00074 | 687 | 57,6 |
| 18,153 | 4,88302 | 78 | 6,5 |
| 19,525 | 4,54288 | 341 | 28,6 |
| 20,343 | 4,362 | 133 | 11,2 |
| 20,795 | 4,26816 | 434 | 36,4 |
| 21,147 | 4,19799 | 429 | 36 |
| 21,647 | 4,10205 | 1192 | 100 |
| 22,548 | 3,94017 | 131 | 11 |
| 23,079 | 3,8507 | 113 | 9,5 |
| 23,59 | 3,76837 | 84 | 7 |
| 23,909 | 3,7188 | 582 | 48,8 |
| 24,584 | 3,6182 | 388 | 32,6 |
| 24,936 | 3,56793 | 284 | 23,8 |
| 25,434 | 3,49919 | 264 | 22,1 |
| 25,913 | 3,4356 | 110 | 9,2 |
| 26,684 | 3,33805 | 229 | 19,2 |
| 27,275 | 3,26701 | 246 | 20,6 |
| 27,688 | 3,21925 | 135 | 11,3 |
| 29,14 | 3,06206 | 81 | 6,8 |
| 29,552 | 3,02031 | 141 | 11,8 |
| 29,74 | 3,00164 | 144 | 12,1 |
| 30,247 | 2,95244 | 393 | 33 |
| 30,877 | 2,89362 | 105 | 8,8 |
| 31,519 | 2,83616 | 150 | 12,6 |
| 31,86 | 2,8066 | 66 | 5,5 |
| 32,559 | 2,7479 | 93 | 7,8 |
| 32,935 | 2,7174 | 118 | 9,9 |
| 33,356 | 2,68406 | 143 | 12 |
| 33,854 | 2,6457 | 74 | 6,2 |
| 34,26 | 2,61527 | 89 | 7,5 |
| 34,57 | 2,5925 | 120 | 10,1 |
| 35,183 | 2,54873 | 96 | 8,1 |
| 35,914 | 2,49852 | 63 | 5,3 |
| 36,364 | 2,46864 | 92 | 7,7 |
| 36,881 | 2,43516 | 94 | 7,9 |
| 37,193 | 2,41545 | 76 | 6,4 |
| 38,662 | 2,327 | 134 | 11,2 |
| 39,672 | 2,27409 | 92 | 7,7 |

According to analysis results, crystalline 1,4-butanediol solvate of tiotropium bromide is characterized by giving peaks at a 2θ angle of 21.6 ± 0.2° in X-ray powder diffraction pattern.

### - DSC analysis results

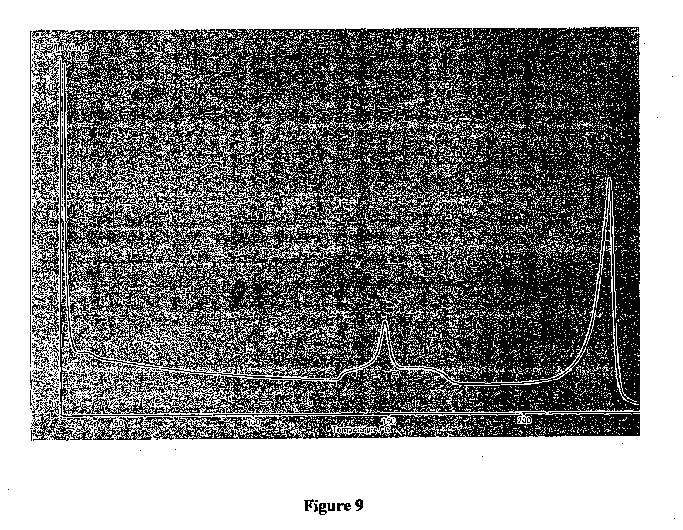

DSC thermogram of crystalline 1,4-butandiol solvate of tiotropium bromide is given in Figure 9.

According to analysis results, crystalline 1,4-butanediole solvate of tiotropium bromide is characterized by giving an endotermic peak in the range of 130 to 180°C in DSC thermogram.

### Preparation processes of solvates in accordance with the invention

### • Crystalline allyl alcohol solvate of tiotropium bromide

Preparation process of crystalline allyl alcohol solvate of tiotropium bromide is characterised by comprising the steps of dissolving tiotropium bromide in allyl alcohol preferably at a temperature in the range of 40-60°C, more preferably at 50°C; cooling the solution below 35°C, preferably to 20°C; adding diethyl ether and pentane respectively to the solution; filtering the solution preferably by cooling, more preferably by cooling to 0°C; and washing and drying crystals obtained thereby with pentane.

### • Crystalline furfuryl alcohol solvate of tiotropium bromide

Preparation process of crystalline furfuryl alcohol solvate of tiotropium bromide is characterized by comprising the steps of dissolving tiotropium bromide in furfuryl alcohol; adding diethyl ether, to the solution; and after filtering the precipitation, washing the solution with diethyl ether, and leaving it to dry.

### • Crystalline 1,4-butanediol solvate of tiotropium bromide

Preparation process of crystalline 1,4-butandiol solvate of tiotropium bromide is characterized by comprising the steps of dissolving tiotropium bromide monohydrate by boiling in a mixture of acetone and 1,4-butanediol; precipitating the solution by adding and mixing acetone and dietyl ether; and after filtering the solution, washing the solution with diethyl ether, and leaving it to dry.

### Examples for preparation processes of solvates in accordance with invention

### • Crystalline allyl alcohol solvate of tiotropium bromide

353 mg of tiotropium bromide is dissolved in 40 ml of allyl alcohol at 50°C. After cooling the solution to 20°C, 150 ml of diethyl ether and 50 ml of pentane are added to the solution respectively. Solution is cooled to 0°C. Solution mixed for a while, is filtered after cooling. Crystals obtained thereby are washed with pentane and dried.

### • Crystalline furfuryl alcohol solvate of tiotropium bromide

272.7 mg of tiotropium bromide is dissolved in 10 ml furfuryl alcohol at room temperature. Diethyl ether is added to the solution by mixing with 10 seconds intervals (7 x 10ml). After filtering the precipitate, the solution is washed with diethyl ether (2 x 10 ml) and left to dry.

### • Crystalline 1,4-butandiol solvate of tiotropium bromide

776 mg of tiotropium bromide monohydrate is dissolved and by boiling in a mixture of 20 ml of acetone and 8 ml of 1,4-butanediole. 18ml of acetone and 100 ml of diethyl ether are added to the solution and it is mixed and precipitated. After filtering the solution, it is washed with 20 ml of ether and left to dry.

### Pharmaceutical compositions comprising solvates in accordance with the invention

Crystalline solvates of tiotropium bromide in accordance with the invention are forms which have better structural properties to meet the requirements with regard to inhalation. The term "crystalline solvates of tiotropium bromide in accordance with the invention" is related to crystalline allyl alcohol solvate, furfuryl alcohol solvate and 1,4-butanediol solvate of tiotropium bromide. Said forms should be formulated for use in the treatment of inhalation route. Pharmaceutical compositions comprising a pharmaceutically acceptable, non-toxic and therapeutically effective amount of new crystalline solvates of tiotropium bromide in accordance with the invention and their preparation methods are properties of the invention. Pharmaceutical compositions containing crystalline solvates of tiotropium bromide in accordance with the invention, are in the form of dry powder or pressurized metered dose inhalation composition, preferably in the form of dry powder inhalation composition.

During the preparation of dry powder inhalation composition, there are two methods which are widely applied for delivery of drug in required amount to target area. One of them is the controlled agglomeration of non-diluted drug; the other is based on the adhesion of micronized drug particles to surface of inert carrier of large particle size. Pharmaceutical compositions in accordance with the invention are prepared by using preferably the second method. When the second method is used, pharmaceutical composition comprises at least one pharmaceutically acceptable inert carrier and optionally at least one pharmaceutically acceptable excipient different from carrier(s), along with the active ingredient.

The term "micronized drug particles" means crystalline allyl alcohol solvate, furfuryl alcohol solvate and 1,4-butanediole solvate of tiotropium bromide. Crystalline solvates of tiotropium bromide in accordance with the invention are characterized by having average particle size ranging from 1 to 10 µm, preferably from 1 to 5 µm. Pharmaceutical compositions in accordance with the invention are characterized by containing crystalline solvates of tiotropium bromide in the proportion of 0.001-50%, preferably 0.01-10%.

The term "inert carrier" means a carrier which is preferably lactose, more preferably lactose monohydrate for dry powder inhalation composition in accordance with the invention. Pharmaceutical compositions in accordance with the invention can contain at least one inert carrier of large particle size and of small particle size, and optionally at least one excipient. Inert carrier of large particle size in accordance with the invention is characterized by having an average particle size (d₅₀) ranging from 10 to 250 µm, preferably from 10 to 150 µm, more preferably 150 µm; inert carrier of small particle size is characterized by having an average particle size (d₅₀) ranging from 1 to 10 µm, preferably 10 µm. As carriers of large particle and of small particle can be same materials having different particle size, they can also be different.

At least one pharmaceutically acceptable excipient can be selected from the group consisting of carbohydrates such as lactose, glucose, fructose, galactose, sucrose, maltose, trehalose, maltodextrins, dextrans, cyclodextrins, starch and cellulose; polyalcohols such as sorbitol, mannitol and xylitol; amino acids such as glycine, arginine, lysine, aspartic acid and glutamic acid; peptides such as human serum albumin; gelatin; various salts and taste masking agents. However, said pharmaceutically acceptable excipient is not limited with the ones disclosed above

During the preparation of pressurized metered dose inhalation compositions, two formulation strategies are applied depending on physico-chemical properties of the active ingredient and the pressurized gas system. One of them is the solution formulation, the other is suspension formulation. Preferred pharmaceutical composition comprises propellants, surfactants and at least one essential excipient selected from the group of co-solvents and optionally at least one other pharmaceutically acceptable excipient, along with the active ingredient.

The term "active ingredient" means crystalline allyl alcohol solvate, furfuryl alcohol solvate and 1,4-butanediole solvate of tiotropium bromide. Crystalline solvates of tiotropium bromide in accordance with the invention are characterized by having an average particle size ranging from 1 to 10 µm, preferably from 1 to 5µm. Pharmaceutical compositions in accordance with the invention are characterized by containing crystalline solvates of tiotropium bromide in the proportion of 0.001-50%, preferably 0.01- 10%.

Depending on the formulation strategy, at least one pharmaceutically acceptable excipient can be selected from the group consisting of propellants such as chlorofluorocarbons, hydrofluoroalkanes and hydrocarbons; surfactants such as oleic acid, polysorbates, propylene glycole, polyethylene glycol, cetyl alcohol, stearyl alcohol, sorbitan fatty acid esters, sugar esters of fatty acid esters, glycerides of fatty acids, isopropyl myristate and lecithin; cosolvents such as ethanol, water and diethyl ether; antioxydants such as butylhydroxyanisol (BHA), sodium ascorbate, butylhydroxytoluen (BHT), sodium sulfite, gallates (such as propyl gallate), tocopherol, citric acid, malic acid, ascorbic acid, acetylcysteine, fumaric acid, lecithin, ascorbyl palmitate, ethylendiamine tetraacetate; and sweeteners. However, said pharmaceutically acceptable excipient is not limited with the ones disclosed above.

Pharmaceutical compositions of crystalline solvates of tiotropium bromide in accordance with invention, can be further contain at least one active ingredient selected from the group of other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors. Crystalline solvates of tiotropium bromide in accordance with the invention and at least one active ingredient selected from the said group can be used separately, sequentially and simultaneously.

Another aspect of the invention is the pharmaceutical compositions containing crystalline solvates of tiotropium bromide in accordance with the invention for use in the treatment of respiratory disorders, especially asthma and COPD.

### Preparation methods of pharmaceutical compositions comprising solvates in accordance with the invention

Preparation method of dry powder inhalation compositions in accordance with the invention is characterized by comprising the following steps:
- sieving inert carrier of large particle size and of small particle size separately,
- obtaining the first mixture as a result of adding sieved inert carrier of small particle size to crystalline solvates of tiotropium bromide,
- sieving the resulting first mixture,
- obtaining the second mixture as a result of adding sieved inert carrier of large particle size to the first mixture,
- sieving and mixing the second mixture,
- making ready the final mixture for filling capsules.

Preparation method of pressurized metered dose inhalation compositions in accordance with the invention is characterized by comprising the following steps:
- cooling the production vessel to -25°C
- pumping pressurized gas into the vessel
- adding crystalline solvates of tiotropium bromide to the vessel and mixing resulting mixture
- filling final mixture to appropriate containers.

### Examples for pharmaceutical compositions comprising solvates in accordance with the invention

Examples of pharmaceutical formulations in accordance with the invention are listed below, These examples are given to explain the invention, and this invention is not limited by these examples. Amounts of active agent, defined in formulation examples, belong to pure substance; and required amount of solvate can be calculated by that the proportion of solvent expected to be contained by solvate, is multiplied with the amount of pure substance and obtained result is added to the amount of pure substance.

**Example 1:Formulation of dry powder inhalation**

| Content | Amount(mg) |
|---|---|
| Tiotropium bromide | 0.0225 |
| Lactose monohydrate (d₅₀=150µm) | 19.9500 |
| Lactose monohydrate (d₅₀=10µm) | 5.0275 |
| Total weight | 25 |

**Example 2:Formulation of dry powder inhalation**

| Content | Amount (mg) |
|---|---|
| Tiotropium bromide | 0.0217 |
| Lactose monohydrate (d₅₀=150µm) | 19.9500 |
| Lactose monohydrate (d₅₀=10µm) | 5.0283 |
| Total weight | 25 |

**Example 3: Formulation of pressurized metered dose inhalation**

| Content | Amount (mg) |
|---|---|
| Tiotropium bromide | 0.0225 |
| HFA 134 | 74.98 |
| Total weight | 75.0025 |

**Example 4: Formulation of pressurized metered dose inhalation**

| Content | Amount (mg) |
|---|---|
| Tiotropium bromide | 0.0217 |
| HFA 227 | 74.98 |
| Total weight | 75.0017 |

## Claims

1. Crystalline allyl alcohol solvate of tiotropium bromide

2. Crystalline allyl alcohol solvate of tiotropium bromide according to Claim 1, **characterized by** giving a characteristic peak at a 2θ angle of 21.4 ± 0.2° in X-ray powder diffraction pattern.

3. Crystalline allyl alcohol solvate of tiotropium bromide according to Claim 1, **characterized by** giving an endotermic peak in the range of 130 to 180°C in DSC thermogram.

4. A preparation process of crystalline allyl alcohol solvate, of tiotropium bromide, **characterized by** dissolving tiotropium bromide in allyl alcohol, preferably at a temperature in the range of 40 to 60°C, more preferably at 50°C; cooling the solution below 35°C, preferably to 20°C; adding diethyl ether and pentane respectively to the solution; filtering the solution preferably by cooling, more preferably by cooling to 0°C; and washing and drying crystals obtained thereby, with pentane.

5. Crystalline furfuryl alcohol solvate of tiotropium bromide

6. Crystalline furfuryl alcohol solvate of tiotropium bromide according to Claim 5, **characterized by** giving a characteristic peak at a 2θ angle of 18.2 ± 0.2° in X-ray powder diffraction pattern.

7. Crystalline furfuryl alcohol solvate of tiotropium bromide according to Claim 5, **characterized by** giving an endotermic peak in the range of 150 to 190°C in DSC thermogram.

8. A preparation process of crystalline furfuryl alcohol solvate of tiotropium bromide, **characterized by** dissolving tiotropium bromide in furfuryl alcohol; adding diethyl ether to the solution; and after filtering the precipitate, washing the solution with diethyl ether, and leaving it to dry.

9. Crystalline 1,4-butanediol solvate of tiotropium bromide

10. Crystalline 1,4-butanediole alcohol solvate of tiotropium bromide according to Claim 9, **characterized by** giving a characteristic peak at a 2θ angle of 21.6 ± 0.2° in X-ray powder diffraction pattern.

11. Crystalline 1,4-butanediole alcohol solvate of tiotropium bromide according to Claim 9, **characterized by** giving an endotermic peak in the range of 130 to 180°C in DSC thermogram.

12. Preparation process of crystalline 1,4-butanediole solvate of tiotropium bromide **characterized by** dissolving and boiling tiotropium bromide monohydrate in a mixture of acetone and 1,4-butanediole; precipitating the solution by adding and mixing acetone and dietyl ether; and after filtering the the solution, washing the solution with diethyl ether, and leaving it to dry.

13. Crystalline solvate of tiotropium bromide according to any of claims 1,5 and 9, **characterized by** having an average particle size ranging from 1 to 10 µm, preferably from 1 to 5µm.

14. Pharmaceutical composition **characterized by** containing a crystalline solvate of tiotropium bromide according to any of claims 1, 5 and 9.

15. Pharmaceutical composition according to claim 14, **characterized by** containing a crystalline solvate of tiotropium bromide according to any of claims 1, 5 and 9, in the proportion of 0.001-50%, preferably 0.01-10%.

16. Pharmaceutical composition according to claim 15, **characterized by** being in the form of dry powder or pressurized metered inhalation composition; preferably in the form of dry powder inhalation composition.

17. Pharmaceutical composition according to any of claims 14 to 16, **characterized in that** pharmaceutical composition is further comprising at least one active ingredient selected from the group of other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors for administering separately, sequentially and simultaneously.

18. Pharmaceutical composition according to any of claims 14 to 16, **characterized in that** pharmaceutical composition is used in the treatment of respiratory disorders, especially asthma and COPD.

## Patentansprüche

1. Das kristalline Allylalkohol-Solvat des Tiotropiumbromids

2. Das kristalline Allylalkohol-Solvat des Tiotropiumbromids nach Anspruch 1, **dadurch gekennzeichnet, dass** es bei einem 20 Winkel von 21,4 ± 0,2° bei der Röntgenpulverbeugungsmuster einen charakteristischen Spitzenwert ergibt.

3. Das kristalline Allylalkohol-Solvat des Tiotropiumbromids nach Anspruch 1, **dadurch gekennzeichnet, dass** es eine endotherme Peak im Bereich von 130 bis 180 ° C im DSC-Thermogramm ergibt.

4. Herstellungsverfahren für das kristalline Allylalkohol-Solvat des Tiotropiumbromids, **dadurch gekennzeichnet, dass** es das Tiotropiumbromid im Allylalkohol vorzugsweise bei einer Temperatur im Bereich von 40 bis 60°C, besonders bevorzugt bei 50°C auflöst; die Lösung unter 35°C, vorzugsweise auf 20°C abkühlt; Diethylether und Pentan jeweils zu der Lösung gibt; die Lösung vorzugsweise durch Kühlen, besser durch Kühlen auf 0°C filtriert und die dabei erhaltenen Kristallen mit Pentan wäscht und trocknet.

5. Das kristalline Furfurylalkohol-Solvat des Tiotropiumbromids

6. Das kristalline Furfurylalkohol-Solvat des Tiotropiumbromids nach Anspruch 5, **dadurch gekennzeichnet, dass** es bei einem 2θ Winkel von 18.2 ± 0.2° bei der Röntgenpulverbeugungsmuster einen charakteristischen Spitzenwert ergibt.

7. Das kristalline Furfurylalkohol-Solvat des Tiotropiumbromids nach Anspruch 5, **dadurch gekennzeichnet, dass** es eine endotherme Peak im Bereich von 150 bis 190 ° C im DSC-Thermogramm ergibt.

8. Herstellungsverfahren für das kristalline Furfurylalkohol-Solvat des Tiotropiumbromids, **dadurch gekennzeichnet, dass** es das Tiotropiumbromid im Furfurylalkohol auflöst, Diethylether zu der Lösung gibt und nach dem Filtern des Niederschlags die Lösung mit Diethylether wäscht und es zu trocknen lässt.

9. Das kristalline 1,4-Butandiol-Solvat des Tiotropiumbromids

10. Das kristalline 1,4-Butandiol Alkohol-Solvat des Tiotropiumbromids nach Anspruch 9, **dadurch gekennzeichnet,dass** es bei einem 2θ Winkel von 21,6 ± 0,2 ° bei der Röntgenpulverbeugungsmuster einen charakteristischen Spitzenwert ergibt.

11. Das kristalline 1,4-Butandiol Alkohol-Solvat des Tiotropiumbromids nach Anspruch 9, **dadurch gekennzeichnet, dass** es eine endotherme Peak im Bereich von 130 bis 180 ° C im DSC-Thermogramm ergibt.

12. Herstellungsverfahren von kristallinem 1,4-Butandiol-Solvat des Tiotropiumbromids, **dadurch gekennzeichnet, dass** es das Tiotropiumbromid-Monohydrat in einem Gemisch aus Aceton und 1,4-Butandiol auflöst und siedet; die Lösung durch Zugabe und Mischen von Aceton und Diethylether niederschlägt und nach dem Filtern der Lösung die Lösung mit Diethylether wäscht und es zu trocknen lässt.

13. Das kristalline Solvat des Tiotropiumbromids nach einem der Ansprüche 1,5 und 9, **dadurch gekennzeichnet, dass** es eine mittlere Teilchengröße im Bereich von 1 bis 10 µm, vorzugsweise von 1 bis 5pm hat.

14. Pharmazeutische Zusammensetzung, **dadurch gekennzeichnet, dass** sie ein kristallines Solvat des Tiotropiumbromids nach einem der Ansprüche 1, 5 und 9 beinhaltet.

15. Pharmazeutische Zusammensetzung nach Anspruch 14, **dadurch gekennzeichnet, dass** sie nach einem der Ansprüche 1, 5 und 9 ein kristallines Solvat des Tiotropiumbromids im Verhältnis von 0,001 bis 50%, vorzugsweise 0,01-10% beinhaltet

16. Pharmazeutische Zusammensetzung nach Anspruch 15, **dadurch gekennzeichnet, dass** sie in Form eines trockenen Pulvers oder einer gezwungen, gemessenenlnhalationszusammensetzung ist; bevorzugt in Form einer Trockenpulver-Inhalationszusammensetzung.

17. Pharmazeutische Zusammensetzung nach einem der Ansprüche 14 bis 16, **dadurch gekennzeichnet**, dassdie pharmazeutische Zusammensetzung ferner wenigstens einen Wirkstoff umfasst , der aus der Gruppe von anderen anticholinergen Mitteln, adrenergen Agonisten, antiallergischen Mitteln, entzündungshemmenden Mitteln, Antihistaminen, Steroiden, Leukotrienrezeptor-Antagonisten, antimuskarinischen Wirkstoffen, PDE-Inhibitoren und EGFR-Inhibitoren für die getrennte, sequenzielle und simultane Verwaltung ausgewählt wird.

18. Pharmazeutische Zusammensetzung nach einem der Ansprüche 14 bis 16, **dadurch gekennzeichnet, dass** pharmazeutische Zusammensetzung bei der Behandlung von Atemwegserkrankungen, insbesondere von Asthma und COPD verwendet wird.

## Revendications

1. Solvate cristallin d'alcool allylique de bromure de tiotropium.

2. Solvate cristallin d'alcool allylique de bromure de tiotropium selon la revendication 1, **caractérisé par** donnant un pic à un angle 2θ de 21.4 ± 0.2° dans un diagramme de diffraction des rayons X de poudre.

3. Solvate cristallin d'alcool allylique de bromure de tiotropium selon la revendication 1, **caractérisé par** donnant un pic endothermique dans la plage de 130°C à 180 °C dans un thermogramme DSC.

4. Un procédé de préparation du solvate cristallin d'alcool allylique de bromure de tiotropium, **caractérisé par** dissolvant le bromure de tiotropium dans l'alcool allylique, de préférence à une température dans la plage de 40°C à 60°C, de manière plus préférée à 50°C; refroidissant la solution au-dessous de 35°C, de préférence à 20°C; ajoutant d'éther diéthylique et de pentane respectivement à la solution; filtrant la solution de préférence par refroidissement, de manière plus préférée par refroidissement à 0°C; et lavant et séchant les cristaux ainsi obtenus avec pentane.

5. Solvate cristallin d'alcool furfurylique de bromure de tiotropium.

6. Solvate cristallin d'alcool furfurylique de bromure de tiotropium selon la revendication 5, **caractérisé par** donnant un pic à un angle 2θ de 18.2 ± 0.2° dans un diagramme de diffraction des rayons X de poudre.

7. Solvate cristallin d'alcool furfurylique de bromure de tiotropium selon la revendication 5, **caractérisé par** donnant un pic endothermique dans la plage de 150°C à 190 □ dans un thermogramme DSC.

8. Un procédé de préparation du solvate cristallin d'alcool furfurylique de bromure de tiotropium, **caractérisé par** dissolvant le bromure de tiotropium dans l'alcool furfurylique; ajoutant d'éther diéthylique à la solution; et après filtrant le précipité, lavant la solution avec l'éther diéthylique, et laissant le sécher.

9. Solvate cristallin de butane-1,4-diol de bromure de tiotropium.

10. Solvate cristallin de butane-1,4-diol de bromure de tiotropium selon la revendication 9, **caractérisé par** donnant un pic à un angle 20 de 21.6 ± 0.2° dans un diagramme de diffraction des rayons X de poudre.

11. Solvate cristallin de butane-1,4-diol de bromure de tiotropium selon la revendication 9, **caractérisé par** donnant un pic endothermique dans la plage de 130°C à 180°C dans un thermogramme DSC.

12. Un procédé de préparation du solvate cristallin de butane-1,4-diol de bromure de tiotropium **caractérisé par** dissolvant et bouillant le bromure de tiotropium monohydraté dans un mélange d'acétone et butane-1,4-diol ; précipitant la solution par ajoutant et mélangeant d'acétone et d'éther diéthylique; et après filtrant la solution, lavant la solution avec éther diéthylique, et laissant le sécher.

13. Un solvate cristallin de bromure de tiotropium selon quelconque des revendications 1, 5 et 9, **caractérisé par** ayant une taille moyenne des particules dans la plage de 1 à 10 µm, de préférence de 1 à 5µm.

14. Composition pharmaceutique **caractérisée par** contenant un solvate cristallin de bromure de tiotropium selon quelconque des revendications 1, 5 et 9.

15. Composition pharmaceutique selon la revendication 14, **caractérisée par** contenant un solvate cristallin de bromure de tiotropium selon quelconque des revendications 1, 5 et 9, dans la proportion de 0.001 -50%, de préférence 0.01-10%.

16. Composition pharmaceutique selon la revendication 15, **caractérisée par** étant dans la forme d'un poudre sèche ou une composition d'inhalation de doseur pressurisé; de préférence dans la forme d'une composition d'inhalation de poudre sèche.

17. Composition pharmaceutique selon quelconque des revendications 14 à 16, **caractérisée en ce que** la composition pharmaceutique comprend de plus au moins un élément actif choisi entre le group des autres agents anti-cholinergiques, les agonistes adrénergiques, les agents antiallergiques, les agents anti-inflammatoires, les antihistaminiques, les stéroïdes, les antagonistes des leucotriènes des récepteurs, les agents anti-muscariniques, les inhibiteurs PDE et les inhibiteurs EGFR pour administration séparément, par séquence et simultanément.

18. Composition pharmaceutique selon quelconque des revendications 14 à 16, **caractérisée en ce que** la composition pharmaceutique est utilisée dans le traitement des désordres respiratoires, surtout l'asthme et BPCO.
